(19) Europäisches Patentamt
European Patent Office
Office européen des brevets

(11) Publication number: **0 039 804**
**B1**

(12) # EUROPEAN PATENT SPECIFICATION

(45) Date of publication of patent specification: **20.08.86**

(21) Application number: **81103072.5**

(22) Date of filing: **23.04.81**

(51) Int. Cl.[4]: **C 07 D 217/26,**
C 07 D 223/16,
C 07 D 279/08,
C 07 D 495/04, C 07 D 471/04
// A61K31/395 ,(C07D471/04,
221:00, 209:00),(C07D495/04,
333:00, 221:00)

(54) **Cyclic amides.**

(30) Priority: **12.05.80 US 148083**

(43) Date of publication of application:
**18.11.81 Bulletin 81/46**

(45) Publication of the grant of the patent:
**20.08.86 Bulletin 86/34**

(84) Designated Contracting States:
**AT BE CH DE FR GB IT LI LU NL SE**

(56) References cited:
**EP-A-0 012 845**
**EP-A-0 018 104**
**EP-A-0 029 488**
**EP-A-0 031 741**
**GB-A-2 048 863**
**US-A-4 251 444**

(73) Proprietor: **USV PHARMACEUTICAL CORPORATION**
**1 Scarsdale Road**
**Tuckahoe New York (US)**

(72) Inventor: **Suh, John Taiyoung**
**59 Stanwich Road**
**Greenwich Connecticut (US)**
Inventor: **Williams, Bruce Earl**
**8822 Upper 89th Street Circle South**
**Cottage Grove Minnesota (US)**
Inventor: **Skiles, Jerry Wallace**
**1 Consulate Drive Tuckahoe**
**New York (US)**

(74) Representative: **Patentanwälte Grünecker, Dr. Kinkeldey, Dr. Stockmair, Dr. Schumann, Jakob, Dr. Bezold, Meister, Hilgers, Dr. Meyer-Plath**
**Maximilianstrasse 58**
**D-8000 München 22 (DE)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 039 804 B1

**Description**

This invention relates to chemical compounds of formula (I)

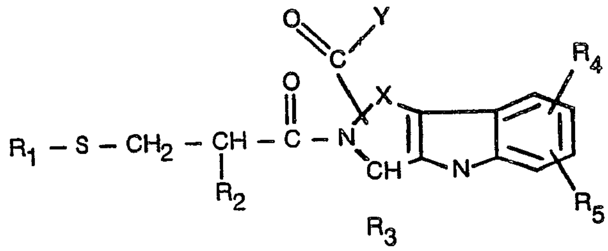

I

wherein
$R_1$ = H, alkanoyl having 1—6 C-atoms in the alkyl moiety
$R_2$ = alkyl having 1—6 C-atoms
$R_3$ = H, alkyl having 1—6 C-atoms
X = $—CH_2—CH_2—$, $—CH_2—CH_2—CH_2—$, —CH=CH—
Y = OH, alkoxy having 1—6 C-atoms
$R_4$, $R_5$ = H, OH, alkoxy having 1—6 C-atoms, halogen and pharmaceutically acceptable salts thereof.

It also relates to the following chemical compounds:
2-(3'-Acetylthio-2'-methylpropanoyl)-1-phenyl-3-carbomethoxy-1,2,3,4-tetrahydro-β-carboline,
2-(3'-Mercapto-2'-methylpropanoyl)-1-phenyl-3-carbomethoxy-1,2,3,4-tetrahydro-β-carboline,
N-(3'-Acetylthio-2'-methylpropanoyl)-1-(3',4'-dimethoxyphenyl)-1,2,3,4-tetrahydroisoquinoline-3-carboxylic acid,
N-(3'Trimethylacetylthio-2'-methylpropanoyl)-1,2,3,4-tetrahydrothieno[3,2-c]-pyridine-3-carboxylic acid,
2',6'-Dichlorobenzoylthio-2'-methylpropanoyl-1,3-benzothiazine-2-carboxylic acid and
N-(2'-Acetylthiopropanoyl)-benzo-2-azacycloheptane-3-carboxylic acid.

Said compounds possess antihypertensive and angiotensin converting enzyme inhibitory activity.

EP—A—12845 (published after the priority date of the present invention) discloses a tetrahydroisoquinoline compound of the formula:

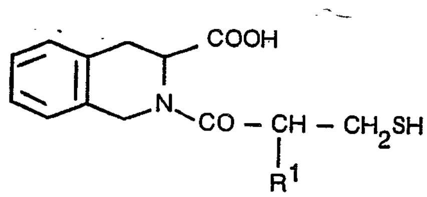

wherein $R^1$ is hydrogen or methyl, or a pharmaceutically acceptable salt thereof.

This compound is useful as a diagnostic or therapeutic agent for angiotensin-related hypertension.

EP—A—18104 (published after the priority date of the present invention discloses tetrahydroisoquinoline derivatives of the formula:

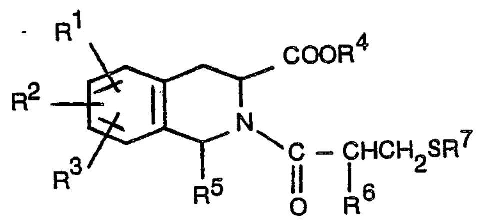

here
wherein $R^1$, $R^2$ and $R^3$, which may be the same or different, each represent hydrogen, halogen, nitro, hydroxyl, lower alkyl, cycloalkyl, lower alkyloxy or cyclic amino, or any two adjacent groups of $R^1$, $R^2$ and $R^3$, when taken together with the ring carbon atoms to which they are attached, form a ring, $R^4$ and $R^5$ each represent hydrogen or lower alkyl, $R^6$ is hydrogen lower alkyl or $—CH_2SR^8$ in which $R^8$ is hydrogen or acyl, $R^7$ is hydrogen or acyl, or $R^7$ and $R^8$ may jointly represent a single bond, when $R^6$ is $—CH_2SR^8$, and their pharmaceutically acceptable salts.

These compounds have inhibitory activities on angiotensin converting enzyme and bradykinin decomposing enzyme, and are useful as antihypertensive agents.

EP—A—29488 and EP—A—31741 refer to compounds related to those of the invention.

The preferred compounds of the invention are those wherein in formula (I) $R_4$ and $R_5$ are hydrogen, Y is hydroxy, $R_2$ is methyl and $R_1$ is hydrogen or acetyl.

It is known to those skilled in the art that those amides of the present invention having an asymmetric carbon atom may exist in racemic or optically active levo or dextro forms. All of these forms are contemplated within the scope of this invention.

The compounds of formula (I) are produced by acylating an amine of the formula (II)

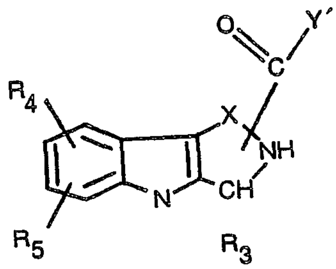

II

wherein

$R_3$, $R_4$, $R_5$ and X are as defined above and

Y' is OM, $OR_6$, $NR_6R_7$ or $—NR_6—(CR_6R_7)_n—CO—Y''$, wherein

M is a pharmaceutically acceptable cation,

$R_6$ and $R_7$ are each hydrogen, alkyl, alkenyl, alkynyl, aryl, heteroaryl, aralkyl, heteroaralkyl, cycloalkyl, polycycloalkyl or heterocycloalkyl,

n is an integer from 0 to 4 and

Y'' is OM, $OR_6$ or $NR_6R_7$ with a mercapto acid or equivalent acylating derivative of the formula (III)

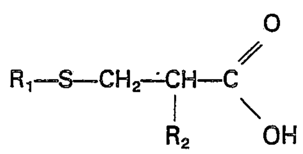

(III)

wherein

$R_1$ and $R_2$ are as defined above, including the optional step of converting the group —COY' to the free acid or to the alkoxy derivatives having 1—6 C-atoms, or optionally derivatizing the product wherein $R_1$ is H to introduce alkanoyl groups having 1—6 C-atoms.

2 - (3' - Acetylthio - 2' - methylpropanoyl - 1 - phenyl - 3 - carbomethoxy - 1,2,3,4 - tetrahydro - β - carboline is produced by reacting 1 - Phenyl - 3 - carbomethoxy - 1,2,3,4 - tetrahydro - β - carboline and 3 - Acetylthio - 2 - methylpropanoyl chloride or other acylating derivatives thereof under acylating conditions.

2 - (3' - Mercapto - 2' - methylpropanoyl) - 1 - phenyl - 3 - carbomethoxy - 1,2,3,4 - tetrahydro - β - carboline is produced by corresponding solvolysis of 2 - (3' - Acetylthio - 2' - methylpropanoyl - 1 - phenyl - 3 - carbomethoxy - 1,2,3,4 - tetrahydro - β - carboline.

A representative procedure for the preparation of compounds of the present invention is outlined in the following amine acylation reaction.

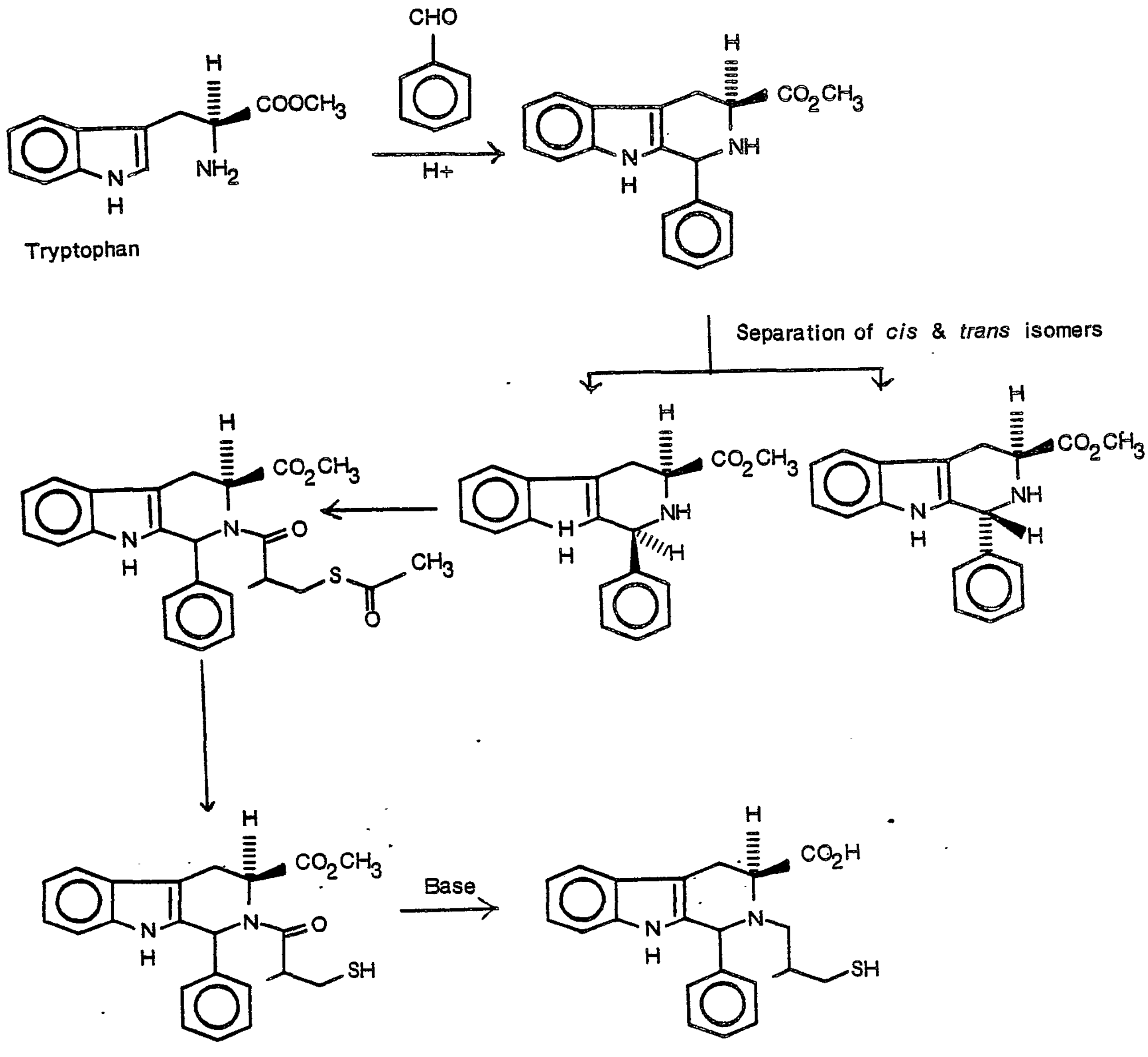


Tryptophan

The desired intermediates may be prepared by well-known reactions.

The invention will be more fully illustrated in the following examples.

Example 1

2-(3'-Acetylthio-2'-methylpropanoyl)-1-phenyl-3-carbomethoxy-1,2,3,4-tetrahydro-β-carboline

1-Phenyl-3-carbomethoxy-1,2,3,4-tetrahydro-β-carboline (10 g, 0.0327 mole) and triethylamine (4.1 g, 0.04 mole) were dissolved in acetonitrile (250 ml) and the resulting mixture was chilled in an ice bath. 3-Acetylthio-2-methylpropanoyl chloride (6.5 g, 0.036 mole) was added portionwise. After all the acid chloride had been added the ice bath was removed and the mixture was stirred at room temperature under nitrogen for two and a half hours. The solvent was evaporated and the residue was dissolved in chloroform. The chloroform was washed with water, 5% aqueous NaOH, 5% aqueous HCl, and water. The chloroform was dried over magnesium sulfate, filtered and evaporated to give the crude product as a lightly colored oil. The product was further purified by chromatography on silica-gel ($CH_2Cl_2$) to give the pure product as a colorless oil (9.6 g, 65%).

Example 2

2-(3'-Mercapto-2'-methylpropanoyl)-1-phenyl-3-carbomethoxy-1,2,3,4-tetrahydro-β-carboline

2 - (3' - Acetylthio - 2' - methylpropanoyl) - 1 - phenyl - 3 - carbomethoxy - 1,2,3,4 - tetrahydro - β - carboline (2 g, 0.0045 mole) was dissolved in methanol (75 ml) and anhydrous ammonia was bubbled through the solution for 15 minutes. The reaction was placed under nitrogen and stirring was continued at room temperature for one hour. The methanol was evaporated and the residue was applied to a cation

exchange column (methanol). The methanol was evaporated and the residue was dissolved in chloroform, washed with water, dried over magnesium sulfate, filtered and evaporated to give the product as a colorless oil (1.6 g, 88%).

Following the procedures described, the following compounds were prepared:

2-(3'-Acetylthio-2'-methylpropanoyl)-6-fluoro-1,2,3,4-tetrahydro-β-carboline-3-carboxylic acid
2-(3'-mercapto-2'-methylpropanoyl)-1-methyl-3-1,2,3,4-tetrahydro-β-carboline-3-carboxylic acid
N-(3'-Acetylthio-2'-methylpropanoyl)-1-(3'4'-dimethoxyphenyl)-1,2,3,4-tetrahydroisoquinoline-3-carboxylic acid
N-(3'-Trimethylacetylthio-2'-methylpropanoyl)-1,2,3,4-tetrahydrothieno[3,2-C]-pyridine-3-carboxylic acid
2'6'-Dichlorobenzoylthio-2'-methylpropanoyl-1,3-benzothiazine-2-carboxylic acid
N-(2'-Acetylthiopropanoyl)-benzo-2-azacycloheptane-3-carboxylic acid

The compounds of the present invention exhibit potent angiotensin enzyme inhibitory activity. Such activity indicates that the compounds of the present invention are useful in the treatment of hypertension. When administered intraperitoneally to spontaneously hypertensive rats the compounds of the present invention effected a reduction in the blood pressure of 20 to 30% at a range of about 100 mg/kg.

The compounds of the present invention may be administered orally or parenterally in the treatment of hypertension, and it will be within the professional judgement and skill of the practitioner to determine the exact amount to be administered.

**Claims for the Contracting States: BE CH DE FR GB IT LI LU NL SE**

1. Compounds of formula (I)

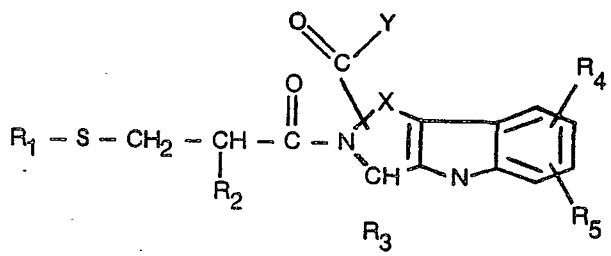

I

wherein

$R_1$ = H, alkanoyl having 1—6 C-atoms in the alkyl moiety
$R_2$ = alkyl having 1—6 C-atoms
$R_3$ = H, alkyl having 1—6 C-atoms
X = $-CH_2-CH_2-$, $-CH_2-CH_2-CH_2-$, $-CH=CH-$
Y = OH, alkoxy having 1—6 C-atoms
$R_4$, $R_5$ = H, OH, alkoxy having 1—6 C-atoms, halogen and pharmaceutically acceptable salts thereof.

2. 2 - (3' - Acetylthio - 2' - methylpropanoyl) - 1 - phenyl - 3 - carbomethoxy - 1,2,3,4 - tetrahydro - β - carboline and 2 - (3' - Mercapto - 2' - methylpropanoyl) - 1 - phenyl - 3 - carbomethoxy - 1,2,3,4 - tetrahydro - β - carboline.

3. N - (3' - Acetylthio - 2' - methylpropanoyl) - 1 - (3',4' - dimethoxyphenyl) - 1,2,3,4 - tetrahydroisoquinoline - 3 - carboxylic acid.

4. N - (3' - Trimethylacetylthio - 2' - methylpropanoyl) - 1,2,3,4 - tetrahydrothieno[3,2 - c] - pyridine - 3 - carboxylic acid.

5. 2',6' - Dichlorobenzoylthio - 2' - methylpropanoyl - 1,3 - benzothiazine - 2 - carboxylic acid.

6. N - (2' - Acetylthiopropanoyl) - benzo - 2 - azacycloheptane - 3 - carboxylic acid.

7. A process for producing the compounds according to claim 1 which comprises acylating an amine of the formula (II)

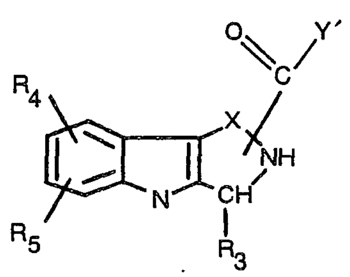

II

wherein

$R_3$, $R_4$, $R_5$ and X are as defined in claim 1 and
Y' is OM, $OR_6$, $NR_6R_7$ or $-NR_6-(CR_6R_7)_n-CO-Y''$, wherein

M is a pharmaceutically acceptable cation,

$R_6$ and $R_7$ are each hydrogen, alkyl, alkenyl, alkynyl, aryl, heteroaryl, aralkyl, heteroaralkyl, cycloalkyl, polycycloalkyl or heterocycloalkyl,

n is an integer from 0 to 4 and

Y″ is OM, $OR_6$ or $NR_6R_7$ with a mercapto acid or equivalent acylating derivative of the formula (III)

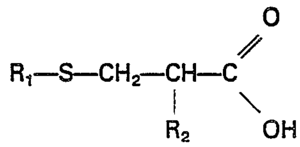

(III)

wherein

$R_1$ and $R_2$ are as defined in claim 1, including the optional step of converting the group —COY′ to the free acid or to the alkoxy derivatives having 1—6 C-atoms, or optionally derivatizing the product wherein $R_1$ is H to introduce alkanoyl groups having 1—6 C-atoms.

8. A process for producing the 2 - (3′ - Acetylthio - 2′ - methylpropanoyl - 1 - phenyl - 3 - carbomethoxy - 1,2,3,4 - tetrahydro - β - carboline of claim 2 which comprises reacting 1 - Phenyl - 3 - carbomethoxy - 1,2,3,4 - tetrahydro - β - carboline and 3 - Acetylthio - 2 - methylpropanoyl chloride or other acylating derivatives thereof under acylating conditions.

9. A process for producing the 2 - (3′ - Mercapto - 2′ - methylpropanoyl) - 1 - phenyl - 3 - carbomethoxy - 1,2,3,4 - tetrahydro - β - carboline of claim 2 which comprises corresponding solvolysis of 2 - (3′ - Acetylthio - 2′ - methylpropanoyl - 1 - phenyl - 3 - carbomethoxy - 1,2,3,4 - tetrahydro - β - carboline.

**Claims for the Contracting State: AT**

1. A process for producing the compounds of formula (I)

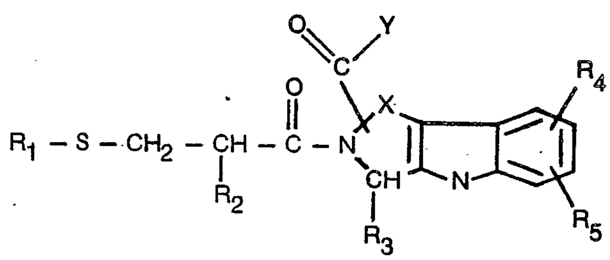

I

wherein

$R_1$ = H, alkanoyl having 1—6 C-atoms in the alkyl moiety

$R_2$ = alkyl having 1—6 C-atoms

$R_3$ = H, alkyl having 1—6 C-atoms

X = $-CH_2-CH_2-$, $-CH_2-CH_2-CH_2-$, $-CH=CH-$

Y = OH, alkoxy having 1—6 C-atoms

$R_4$, $R_5$ = H, OH, alkoxy having 1—6 C-atoms, halogen and pharmaceutically acceptable salts thereof, which comprises acylating an amine of the formula (II)

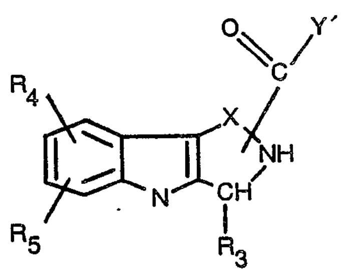

II

wherein

$R_3$, $R_4$, $R_5$ and X are as defined above and

Y′ is OM, $OR_6$, $NR_6R_7$ or $-NR_6-(CR_6R_7)_n-CO-Y''$, wherein

M is a pharmaceutically acceptable cation,

$R_6$ and $R_7$ are each hydrogen, alkyl, alkenyl, alkynyl, aryl, heteroaryl, aralkyl, heteroaralkyl, cycloalkyl, polycycloalkyl or heterocycloalkyl,

n is an integer from 0 to 4 and
Y″ is OM, $OR_6$ or $NR_6R_7$ with a mercapto acid or equivalent acylating derivative of the formula (III)

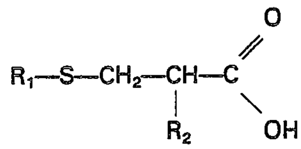

(III)

wherein

$R_1$ and $R_2$ are defined above including the optional step of converting the group —COY′ to the free acid or to the alkoxy derivatives having 1—6 C-atoms, or optionally derivatizing the product wherein $R_1$ is H to introduce alkanoyl groups having 1—6 C-atoms.

2. A process for producing 2 - (3′ - Acetylthio - 2′ - methyl - propanoyl) - 1 - phenyl - 3 - carbomethoxy - 1,2,3,4 - tetrahydro - β - carboline which comprises reacting 1 - Phenyl - 3 - carbomethoxy - 1,2,3,4 - tetrahydro - β - carboline and 3 - Acetylthio - 2 - methylpropanoyl chloride or other acylating derivatives thereof under acylating conditions.

3. A process for producing 2 - (3′ - Mercapto - 2′ - methylpropanoyl) - 1 - phenyl-3-carbomethoxy - 1,2,3,4 - tetrahydro - β - carboline which comprises corresponding solvolysis of 2 - (3′ - Acetylthio - 2′ - methylpropanoyl - 1 - phenyl - 3 - carbomethoxy - 1,2,3,4 - tetrahydro - β - carboline.

**Patentansprüche für die Vertragsstaaten: BE CH DE FR GB IT LI LU NL SE**

1. Verbindungen der Formel (I)

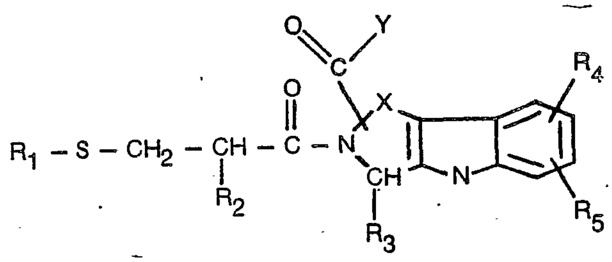

, I

worin

$R_1$ = H, Alkanoyl mit 1 bis 6 C-Atomen in dem Alkylrest,
$R_2$ = Alkyl mit 1 bis 6 C-Atomen,
$R_3$ = H, Alkyl mit 1 bis 6 C-Atomen,
X = $—CH_2—CH_2—$, $—CH_2—CH_2—CH_2—$, —CH=CH—,
Y = OH, Alkoxy mit 1 bis 6 C-Atomen,
$R_4$, $R_5$ = H, OH, Alkoxy mit 1 bis 6 C-Atomen, Halogen und pharmazeutisch annehmbare Salze davon.

2. 2 - (3′ - Acetylthio - 2′ - methylpropanoyl) - 1 - phenyl - 3 - carbomethoxy - 1;2,3,4 - tetrahydro - β - carbolin und 2 - (3′ - Mercapto - 2′ - methylpropanoyl) - 1 - phenyl - 3 - carbomethoxy - 1,2,3,4 - tetrahydro - β - carbolin.

3. N - (3′ - Acetylthio - 2′ - methylpropanoyl) - 1 - (3′,4′ - dimethoxyphenyl) - 1,2,3,4 - tetrahydroisochinolin - 3 - carbonsäure.

4. N - (3′ - Trimethylacetylthio - 2 - methylpropanoyl) - 1,2,3,4 - tetrahydrothieno[3,2 - c] - pyridin - 3 - carbonsäure.

5. 2′6′ - Dichlorbenzoylthio - 2′ - methylpropanoyl - 1,3 - benzothiazin - 2 - carbonsäure.

6. N - (2′ - Acetylthiopropanoyl) - benzo - 2 - azacycloheptan - 3 - carbonsäure.

7. Verfahren zur Herstellung der Verbindungen nach Anspruch 1, welches die Acylierung eines Amins der Formel (II)

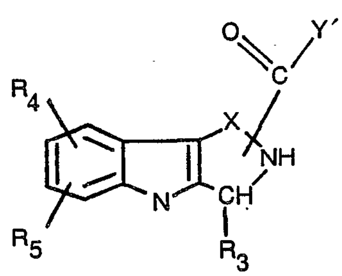

II

worin

$R_3$, $R_4$, $R_5$ und X wie in Anspruch 1 definiert sind und

Y' OM, $OR_6$, $NR_6R_7$ oder $—NR_6—(CR_6R_7)_n—CO—Y''$ ist, worin

M ein pharmazeutisch annehmbares Kation ist,

$R_6$ und $R_7$ jeweils Wasserstoff, Alkyl, Alkenyl, Alkynyl, Aryl, Heteroaryl, Aralkyl, Heteroaralkyl, Cycloalkyl, Polycycloalkyl oder Heterocycloalkyl sind,

n eine ganze Zahl von 0 bis 4 ist und

Y'' OM, $OR_6$ oder $NR_6R_7$ ist, mit einer Mercaptosäure oder einem äquivalenten acylierenden Derivat der Formel (III)

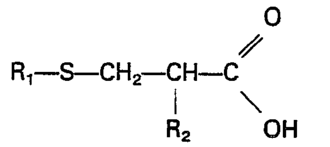

(III)

worin

$R_1$ und $R_2$ wie in Anspruch 1 definiert sind, umfaßt, gegebenenfalls einschließlich einer Stufe der Umwandlung der Gruppe —COY' in die freie Säure oder in die Alkoxyderivate mit 1 bis 6 C-Atomen oder gegebenenfalls die Derivatisierung des Produkts, worin $R_1$ H ist, um Alkanoylgruppen mit 1 bis 6 C-Atomen einzuführen.

8. Verfahren zur Herstellung von 2 - (3' - Acetylthio - 2' - methylpropanoyl) - 1 - phenyl - 3 - carbomethoxy - 1,2,3,4 - tetrahydro - β - carbolin nach Anspruch 2, welches eine Umsetzung von 1 - Phenyl - 3 - carbomethoxy - 1,2,3,4 - tetrahydro - β - carbolin und 3 - Acetylthio - 2 - methylpropanoylchlorid oder anderen acylierenden Derivaten davon unter Acylierungsbedingungen umfaßt.

9. Verfahren zur Herstellung von 2 - (3' - Mercapto - 2' - methylpropanoyl) - 1 - phenyl - 3 - carbomethoxy - 1,2,3,4 - tetrahydro - β - carbolin nach Anspruch 2, welches die entsprechende Solvolyse von 2 - (3' - Acetylthio - 2' - methylpropanoyl) - 1 - phenyl - 3 - carbomethoxy - 1,2,3,4 - tetrahydro - β - carbolin umfaßt.

**Patentansprüche für den Vertragsstaat: AT**

1. Verfahren zur Herstellung der Verbindungen der Formel (I)

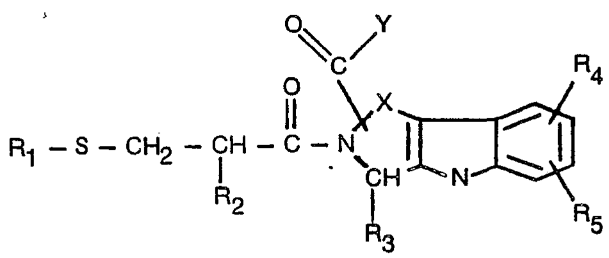

I

worin

$R_1$ = H, Alkanoyl mit 1 bis 6 C-Atomen in dem Alkylrest,

$R_2$ = Alkyl mit 1 bis 6 C-Atomen,

$R_3$ = H, Alkyl mit 1 bis 6 C-Atomen,

X = $—CH_2—CH_2—$, $—CH_2—CH_2—CH_2—$, $—CH=CH—$,

Y = OH, Alkoxy mit 1 bis 6 C-Atomen,

$R_4$, $R_5$ = H, OH, Alkoxy mit 1 bis 6 C-Atomen, Halogen und pharmazeutisch annehmbare Salze davon, welches die Acylierung eines Amins der Formel (II)

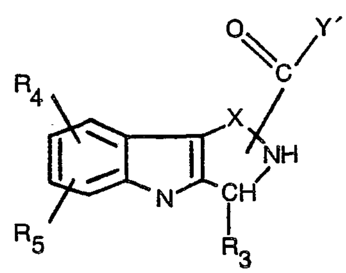

II

worin

$R_3$, $R_4$, $R_5$ und X wie vorstehend definiert sind und

Y′ OM, $OR_6$, $NR_6R_7$ oder $—NR_6—(CR_6R_7)_n—CO—Y''$ ist, worin

M ein pharmazeutisch annehmbares Kation ist,

$R_6$ und $R_7$ jeweils Wasserstoff, Alkyl, Alkenyl, Alkynyl, Aryl, Heteroaryl, Aralkyl, Heteroaralkyl, Cycloalkyl, Polycycloalkyl oder Heterocycloalkyl sind,

n eine ganze Zahl von 0 bis 4 ist und

Y″ OM, $OR_6$ oder $NR_6R_7$ ist, mit einer Mercaptosäure oder einem äquivalenten acylierenden Derivat der Formel (III)

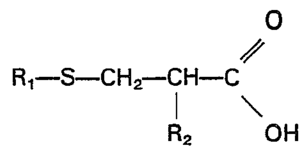

(III)

worin

$R_1$ und $R_2$ wie vorstehend definiert sind, umfaßt, gegebenenfalls einschließlich einer Stufe der Umwandlung der Gruppe —COY′ in die freie Säure oder in die Alkoxyderivate mit 1 bis 6 C-Atomen oder gegebenenfalls die Derivatisierung des Produkts, worin $R_1$ H ist, um Alkanoylgruppen mit 1 bis 6 C-Atomen einzuführen.

2. Verfahren zur Herstellung von 2 - (3′ - Acetylthio - 2′ - methylpropanoyl) - 1 - phenyl - 3 - carbomethoxy - 1,2,3,4 - tetrahydro - β - carbolin, welches eine Umsetzung von 1 - Phenyl - 3 - carbomethoxy - 1,2,3,4 - tetrahydro - β - carbolin und 3 - Acetylthio - 2 - methylpropanoylchlorid oder anderen acylierenden Derivaten davon unter Acylierungsbedingungen umfaßt.

3. Verfahren zur Herstellung von 2 - (3′ - Mercapto - 2′ - methylpropanoyl) - 1 - phenyl - 3 - carbomethoxy - 1,2,3,4 - tetrahydro - β - carbolin, welches die entsprechende Solvolyse von 2 - (3′ - Acetylthio - 2′ - methylpropanoyl) - 1 - phenyl - 3 - carbomethoxy - 1,2,3,4 - tetrahydro - β - carbolin umfaßt.

**Revendications pour les Etats contractants: BE CH DE FR GB IT LI LU NL SE**

1. Composé de formule (I)

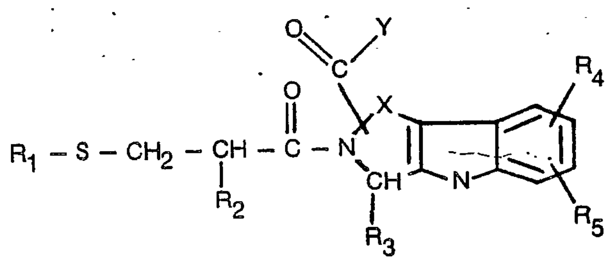

I

formule dans laquelle:

$R_1$ = H, alcanoyle renfermant 1—6 atomes de carbone dans la fraction alkyle

$R_2$ = alkyle renfermant 1—6 atomes de carbone

$R_3$ = H, alkyle renfermant 1—6 atomes de carbone

X = $—CH_2—CH_2—$, $—CH_2—CH_2—CH_2—$, $—CH{=}CH—$

Y = OH, alcoxy renfermant 1—6 atomes de carbone

$R_4$, $R_5$ = H, OH, alcoxy renfermant 1—6 atomes de carbone, halogène, et leurs sels pharmaceutiquement acceptables.

2. (Acétylthio-3′ méthyl-2′ propanoyl)-2-phényl-1 carbométhoxy-3 tétrahydro-1,2,3,4 β-carboline, et (mercapto-3′ méthyl-2′ propanoyl)-2 phényl-1 carbométhoxy-3 tétrahydro-1,2,3,4 β-carboline.

3. Acide N-(acétylthio-3′ méthyl-2′ propanoyl) (diméthoxy-3′,4′ phényl)-1 tétrahydro-1,2,3,4 isoquinoline carboxylique-3.

4. Acide N-(triméthylacétylthio-3′ méthyl-2′ propanoyl) tétrahydro-1,2,3,4 thiéno[3,2-c]-pyridine carboxylique-3.

5. Acide dichloro-2′,6′ benzoylthio méthyl-2′ propanoyl benzothiazine-1,3 carboxylique-2.

6. Acide N-(acétylthio-2′ propanoyl)-benzo-2-azacycloheptane carboxylique-3.

7. Procédé de préparation des composés selon la revendication 1, qui consiste à acyler une amine de formule (II)

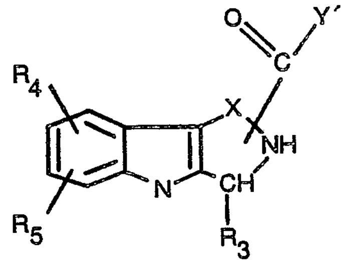

II

formule dans laquelle:

$R_3$, $R_4$,$R_5$ et X sont tels que définis à la revendication 1, et

Y' est OM, $OR_6$, $NR_6R_7$ ou $—NR_6—(CR_6—R_7)_n—CO—Y''$, formules dans lesquelles:

M est un cation pharmaceutiquement acceptable,

$R_6$ et $R_7$ sont chacun hydrogène, alkyle, alcényle, alcynyle, aryle, hétéroaryle, aralkyle, hétéroalkyle, cycloalkyle, polycycloalkyle ou hétérocycloalkyle,

n est un nombre entier allant de 0 à 4, et

Y'' est OM, $OR_6$ ou $NR_6R_7$, avec un mercaptoacide ou un dérivé acylant équivalent de formule (III)

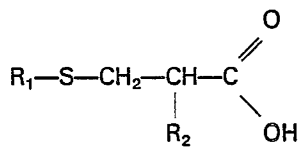

(III)

formule dans laquelle $R_1$ et $R_2$ sont tels que définis à la revendication 1, y compris l'étape facultative de conversion du groupe —COY' en l'acide libre ou en les dérivés alcoxy renfermant 1—6 atomes de carbone, ou facultativement à transformer en un dérivé le produit dans lequel $R_1$ est H, pour introduire des groupes alcanoyle renfermant 1—6 atomes de carbone.

8. Procédé de préparation de l'(acétylthio-3' méthyl-2' propanoyl)-2 phényl-1 carbométhoxy-3 tétrahydro-1,2,3,4 β-carboline de la revendication 2, qui consiste à faire réagir la phényl-1 carbométhoxy-3 tétrahydro-1,2,3,4 β-carboline et le chlorure d'acétylthio-3 méthyl-2 propanoyl ou d'autres dérivés acylants de celui-ci, dans des conditions acylantes.

9. Procédé de préparation de la (mercapto-3' méthyl-2' propanoyl)-2 phényl-1 carbométhoxy-3 tétrahydro-1,2,3,4 β-carboline de la revendication 2, qui consiste à réaliser la solvolyse correspondante de l'(acétylthio-3' méthyl-2' propanoyl)-2 phényl-1 carbométhoxy-3 tétrahydro-1,2,3,4 β-carboline.

**Revendications pour l'Etat contractant: AT**

1. Procédé de préparation des composés de formule

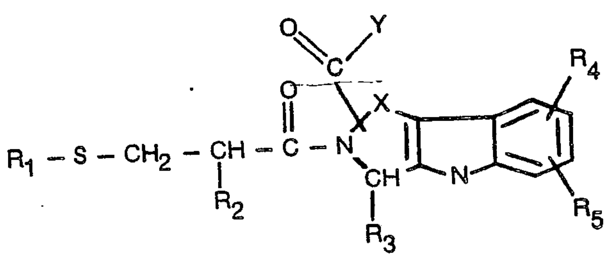

I

formule dans laquelle:

$R_1$ = H, alcanoyle renfermant 1—6 atomes de carbone dans la fraction alkyle

$R_2$ = alkyle renfermant 1—6 atomes de carbone

$R_3$ = H, alkyle renfermant 1—6 atomes de carbone

X = $—CH_2—CH_2—$, $—CH_2—CH_2—CH_2—$, $—CH=CH—$

Y = OH, alcoxy renfermant 1—6 atomes de carbone

$R_4$, $R_5$ = H, OH, alcoxy renfermant 1—6 atomes de carbone, halogène, et de leurs sels pharmaceutiquement acceptables, qui consiste à acyler une amine de formule (II)

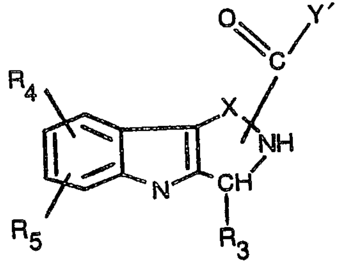

II

formule dans laquelle:

$R_3$, $R_4$, $R_5$ et X sont tels que définis ci-dessus et

Y′ est OM, $OR_6$, $NR_6R_7$ ou $—NR_6—(CR_6R_7)_n—CO—Y''$, formules dans lesquelles:

M est un cation pharmaceutiquement acceptable,

$R_6$ et $R_7$ sont chacun hydrogène, alkyle, alcényle, alcynyle, aryle, hétéroaryle, aralkyle, hétéroaralkyle, cycloalkyle, polycycloalkyle ou hétérocycloalkyle,

n est un nombre entier allant de 0 à 4, et

Y″ est OM, $OR_6$ ou $NR_6R_7$ avec un mercaptoacide ou un dérivé acylant équivalent de formule (III)

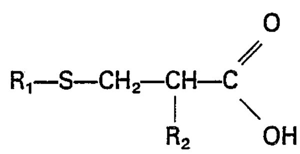

(III)

formule dans laquelle $R_1$ et $R_2$ sont tels que définis ci-dessus, y compris l'étape facultative de conversion du groupe COY′ en l'acide libre ou en les dérivés alcoxy renfermant 1—6 atomes de carbone, ou facultativement à transformer en un dérivé le produit dans lequel $R_1$ est H, pour introduire des groupes alcanoyle renfermant 1—6 atomes de carbone.

2. Procédé de préparation de l'(acétylthio-3′ méthyl-2′ propanoyl)-2 phényl-1 carbométhoxy-3-tétrahydro-1,2,3,4 β-carboline, qui consiste à faire réagir la phényl-1-carboxyméthoxy-3 tétrahydro-1,2,3,4 β-carboline et le chlorure d'acétylthio-3 méthyl-2 propanoyle ou d'autres dérivés acylants de celui-ci, dans des conditions acylantes.

3. Procédé de préparation de la (mercapto-3′ méthyl-2′ propanoyl)-2 phényl-1-carbométhoxy-3 tétrahydro-1,2,3,4 β-carboline, qui consiste à effectuer la solvolyse correspondante de l'(acétylthio-3′ méthyl-2′ propanoyl)-2 phényl-1 carbométhoxy-3 tétrahydro-1,2,3,4 β-carboline.